# EUROPÄISCHE PATENTSCHRIFT

(11) **EP 0 324 174 B1**
(45) Veröffentlichungstag und Bekanntmachung des Hinweises auf die Patenterteilung: **27.10.1993**
(21) Anmeldenummer: 88121826.7
(22) Anmeldetag: 29.12.1988
(51) Int. Cl.: C07D 213/61

(54) **Verfahren zur Herstellung von 2-Chlor-5-methyl-pyridin**
Process for the preparation of 2-chloro-5-methylpyridine
Procédé de préparation de la 2-chloro-5-méthylpyridine

(30) Priorität: 07.01.1988 DE 3800179
(43) Veröffentlichungstag der Anmeldung: 19.07.1989
(73) Patentinhaber: BAYER AG, 51368 Leverkusen (DE)
(72) Erfinder: Gallenkamp, Bernd, Dr., D-5600 Wuppertal 1 (DE); Knops, Hans-Joachim, Dr., D-4019 Monheim (DE)

(56) Entgegenhaltungen:
- CHEMISTRY OF HETEROCYCLIC COMPOUNDS, Band 14, Supplement, Teil 2, Seiten 111-114, Wiley, New York, US; R.A. ABRAMOVITCH et al.: "Pyridine and its Derivatives"
- J. ORG. CHEM., Band 37, Nr. 25, 15. Dezember 1972, Seite 4119, American Chem. Society; R.C. DUTY et al.: "Reactions of Nitrosobenzene, omicron-Nitrosotoluene, and Pyridine N-Oxide with Phosphorus Pentachloride"

## Beschreibung

Die Erfindung betrifft ein neues Verfahren zur Herstellung von 2-Chlor-5-methyl-pyridin.

Es ist bekannt, daß man 2-Chlor-5-methyl-pyridin neben 2-Chlor-3-methyl-pyridin, 4-Chlor-3-methyl-pyridin und 3-Chlor-5-methyl-pyridin erhält, wenn man 3-Methyl-pyridin-1-oxid mit Phosphorylchlorid umsetzt (vgl. Weissberger, Chemistry of Heterocyclic Compounds, Pyridine and its Derivatives, Vol. 14, Supplement, Part 2, S. 112). Hauptprodukt dieser Umsetzung ist 4-Chlor-3-methyl-pyridin; der Anteil an 2-Chlor-5-methyl-pyridin liegt im allgemeinen unter 25 %.

Es wurde nun ein neues Verfahren zur Herstellung von 2-Chlor-5-methyl-pyridin der Formel (I) 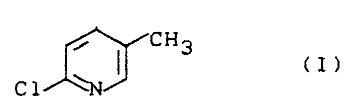
aus 3-Methyl-pyridin-1-oxid der Formel (II) 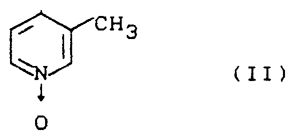
und Phosphorylchlorid gefunden, welches dadurch gekennzeichnet ist, daß man die Umsetzung in Gegenwart einer basischen organischen Stickstoffverbindung der Reihe Dialkylamine, Trialkylamine, Dialkylcycloalkylamine, Dialkylaralkylamine und Dialkylarylamine und in Gegenwart eines inerten organischen Lösungsmittels bei Temperaturen zwischen -50 °C und +50 °C durchführt und das Reaktionsprodukt in üblicher Weise aufarbeitet.

Es ist als überraschend anzusehen, daß gemäß dem erfindungsgemäßen Verfahren unter Verwendung von basischen organischen Stickstoffverbindungen 2-Chlor-5-methylpyridin in erheblich höherer Ausbeute als nach der bisher bekannten Methodik erhalten werden kann, da bei Einsatz von Phosphoryl-chlorid und basischen organischen Stickstoffverbindungen nebeneinander eher mit Nebenprodukten aus der Reaktion dieser Komponenten miteinander zu rechnen war.

Vorteile des erfindungsgemäßen Verfahrens liegen neben der guten Ausbeute am gewünschten Produkt auch darin, daß der Anteil an isomeren Nebenprodukten wesentlich geringer als bei der bisher bekannten Synthesemethode ist. Weiterhin kann man, falls erwünscht, aus dem Reaktionsprodukt die reine Verbindung (I) leicht durch übliche Methoden, zum Beispiel durch destillative Trennung herstellen.

Das erfindungsgemäße Verfahren stellt somit eine wertvolle Bereicherung der Technik dar.

Der Reaktionsablauf beim erfindungsgemäßen Verfahren kann durch das nachstehende Formelschema skizziert werden: 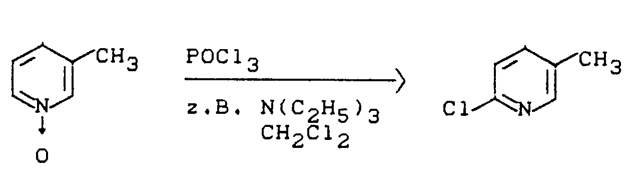
Die Ausgangsstoffe für das erfindungsgemäße Verfahren - 3-Methyl-pyridin-1-oxid und Phosphorylchlorid - sind bekannt (vgl. J. Am. Chem. Soc. 76 (1954), 1286-1291).

Das erfindungsgemäße Verfahren wird in Gegenwart einer basischen Stickstoffverbindung der Reihe Dialkylamine, wie z. B. Diethylamin, Dipropylamin, Diisopropylamin, Dibutylamin, Diisobutylamin und Di-sec-butylamin, Trialkylamine, wie z. B. Triethylamin, Tripropylamin und Tributylamin, Dialkyl-cycloalkylamine, wie z. B. Dimethyl-cyclopentylamin, Diethyl-cyclopentylamin, Dimethyl-cyclohexylamin und Dietyl-cyclohexylamin, Dialkyl-aralkylamine, wie z. B. Dimethyl-benzylamin und Diethyl-benzylamin sowie Dialkylarylamine, wie z. B. Dimethylanilin, durchgeführt.

Diisopropylamin wird als basische organische Stickstoffverbindung besonders bevorzugt.

Das erfindungsgemäße Verfahren wird in Gegenwart eines inerten organischen Lösungsmittels durchgeführt. Hierzu gehören vorzugsweise gegebenenfalls halogenierte Kohlenwasserstoffe, wie Pentan, Hexan, Heptan, Octan, Cyclohexan, Methylcyclohexan, Petrolether, Benzin, Ligroin, Benzol, Toluol, Xylol, Methylenchlorid, Chloroform, Tetrachlormethan, 1,2-Dichlorethan, Chlorbenzol und Dichlorbenzol, Ether, wie Diethylether, Dipropylether, Diisopropylether, Dibutylether, Methyl- tert-butylether, Glycoldimethylether, Diglycoldimethylether, Tetrahydrofuran und Dioxan, Ketone, wie Aceton, Methylethylketon, Diethylketon, Methyl-isopropylketon und Methyl-isobutylketon, Ester, wie Essigsäuremethylester, Essigsäureethylester, Essigsäurepropylester, Essigsäurebutylester und Essigsäureamylester, Nitrile, wie Acetonitril und Propionitril, Amide wie Dimethylformamid, Dimethylacetamid und N-Methyl-pyrrolidon, sowie Dimethylsulfoxid und Sulfolan.

Methylenchlorid wird als Verdünnungsmittel besonders bevorzugt.

Die Reaktionstemperaturen können bei dem erfindungsgemäßen Verfahren in einem größeren Bereich variiert werden. Im allgemeinen arbeitet man bei Temperaturen zwischen -50 °C und +50 °C, vorzugsweise bei Temperaturen zwischen -20 °C und +20 °C.

Das erfindungsgemäße Verfahren wird im allgemeinen unter Normaldruck durchgeführt. Es ist jedoch auch möglich, unter erhöhtem oder vermindertem Druck zwischen 0,1 und 10 bar zu arbeiten.

Zur Durchführung des erfindungsgemäßen Verfahrens setzt man auf 1 Mol 3-Methyl-pyridin-1-oxid der Formel (II) im allgemeinen zwischen 1 und 10 Mol, vorzugsweise zwischen 1,5 und 2,5 Mol, Phosphorylchlorid und ebenfalls zwischen 1 und 10 Mol, vorzugsweise zwischen 1,5 und 2,5 Mol, der basischen organischen Stickstoffverbindung ein. Der Einsatz von angenähert je 2 Mol Phosphorylchlorid und Stickstoffverbindung je Mol 3-Methyl-pyridin-1-oxid wird besonders bevorzugt.

In einer bevorzugten Ausführungsform des erfindungsgemäßen Verfahrens wird das 3-Methyl-pyridin-1-oxid in einem Verdünnungsmittel vorgelegt und unter Rühren und Kühlen das in einem inerten organischen Lösungsmittel gelöste Phosphorylchlorid und die ebenfalls in einem inerten organischen Lösungsmittel gelöste basische organische Stickstoffverbindung gleichzeitig ("simultan", "parallel") eindosiert, wobei vorzugsweise die Zugabe des Phosphorylchlorids mit einem kleinen zeitlichen Vorsprung erfolgt, so daß bereits zwischen 5 % und 20 % des Phosphorylchlorids im Reaktionsgemisch vorliegen, wenn mit der Zugabe der basischen organischen Stickstoffverbindung begonnen wird. Das komplette Reaktionsgemisch wird bis zum Ende der Umsetzung gerührt.

Die Aufarbeitung kann auf übliche Weise durchgeführt werden. Vorzugsweise wird das Reaktionsgemisch unter Rühren und Kühlen mit Wasser versetzt, das organische Lösungsmittel - beispielsweise durch Destillation - entfernt, die wäßrige Phase mit einer wäßrigen Alkalimetall- oder Erdalkalimetall-hydroxidlösung, wie z. B. Natronlauge, auf pH 6 eingestellt und das Reaktionsprodukt daraus weitgehend durch Wasserdampfdestillation entfernt. Der organische Anteil des Wasserdampfdestillats enthält im wesentlichen das Produkt der Formel (I).

Die Reindarstellung der Verbindung der Formel (I) aus dem organischen Anteil des Wasserdampfdestillats kann nach üblichen Methoden, beispielsweise durch eine Feindestillation an einer Füllkörperkolonne erfolgen. Die Verbindung der Formel (I) hat bei einem Druck von 3,3 mb einen Siedepunkt von 56°C. Die Gesamtausbeute bei der Herstellung von reinem Produkt (I) beträgt ausgehend vom 3-Methyl-pyridin-1-oxid 55-62 % der Theorie.

Das nach dem erfindungsgemäßen Verfahren herstellbare 2-Chlor-5-methyl-pyridin ist als Zwischenprodukt für Pharmazeutika bekannt (vgl. DE-OS 28 12 585).

Weiterhin kann 2-Chlor-5-methyl-pyridin als Zwischenprodukt für insektizide Wirkstoffe eingesetzt werden.

### Beispiel 1

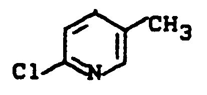

Zu 27,3 g (0,25 Mol) 3-Methyl-pyridin-1-oxid in 400 ml Methylenchlorid läßt man bei ca. -10 °C 10 % einer Lösung von 76,7 g (0,5 Mol) Phosphorylchlorid in 50 ml Methylenchlorid zutropfen. Die restlichen 90 % dieser Lösung werden danach simultan mit einer Mischung von 50,6 g (0,5 Mol) Diisopropylamin in 50 ml Methylenchlorid innerhalb von 3 Stunden bei -10 °C zugetropft. Man rührt ca. 2 1/2 Stunden bei dieser Temperatur nach. Anschließend gibt man unter Kühlung 75 ml Wasser hinzu, destilliert das Methylenchlorid ab, stellt die Reaktionsmischung mit ca. 290 ml 20 %iger Natronlauge auf pH 6 und führt eine Wasserdampfdestillation durch.

Nach üblicher Aufarbeitung erhält man 20,5 g eines Produktgemischs, das nach dem Gaschromatogramm (100 % Methode) folgende Zusammensetzung aufweist:
81 % 2-Chlor-5-methylpyridin,
15 % 2-Chlor-3-methylpyridin, und
1 % β-Picolin.

## Patentansprüche

1. Verfahren zur Herstellung von 2-Chlor-5-methyl-pyridin der Formel (I) 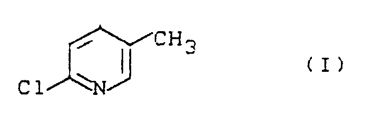 durch Umsetzung von 3-Methyl-pyridin-1-oxid mit Phosphorylchlorid,
dadurch gekennzeichnet, daß man die Umsetzung in Gegenwart einer basischen organischen Stickstoffverbindung der Reihe Dialkylamine, Trialkylamine, Dialkylcycloalkylamine, Dialkylaralkylamine und Dialkylarylamine und in Gegenwart eines inerten organischen Lösungsmittels bei Temperaturen zwischen -50°C und +50°C durchführt und das erhaltene Reaktionsprodukt gegebenenfalls weiter auftrennt.

2. Verfahren nach Anspruch 1, dadurch gekennzeichnet, daß als basische organische Stickstoffverbindung Diisopropylamin eingesetzt wird.

3. Verfahren nach Anspruch 1 und 2, dadurch gekennzeichnet, daß als inertes organisches Lösungsmittel Methylenchlorid eingesetzt wird.

4. Verfahren nach Anspruch 1-3, dadurch gekennzeichnet, daß die Umsetzung bei -20°C bis +20°C durchgeführt wird.

5. Verfahren gemäß Anspruch 1-4, dadurch gekennzeichnet, daß man auf 1 Mol 3-Methyl-pyridin-1-oxid 1 bis 10 Mol der basischen organischen Stickstoffverbindung einsetzt.

6. Verfahren gemäß Anspruch 1-5, dadurch gekennzeichnet, daß man das bei der Umsetzung entstehende Reaktionsgemisch einer Wasserdampfdestillation unterwirft.

7. Verfahren gemäß Anspruch 1-6, dadurch gekennzeichnet, daß man zum Erhalt der reinen Verbindung (I) eine weitere Auftrennung durch Destillation durchführt.

## Claims

1. Process for the preparation of 2-chloro-5-methyl-pyridine of the formula (I) 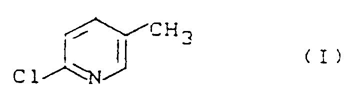 by reaction of 3-methylpyridine 1-oxide with phosphorus oxychloride, characterized in that the reaction is carried out in the presence of a basic organic nitrogen compound from the series consisting of dialkylamines, trialkylamines, dialkylcycloalkylamines, dialkylaralkylamines and dialkylarylamines and in the presence of an inert organic solvent at temperatures between -50°C and +50°C and the resulting reaction product is, if desired, further separated.

2. Process according to Claim 1, characterized in that the basic organic nitrogen compound used is diisopropylamine.

3. Process according to Claim 1 and 2, characterized in that the inert organic solvent used is methylene chloride.

4. Process according to Claim 1-3, characterized in that the reaction is carried out at -20°C to +20°C.

5. Process according to Claim 1-4, characterized in that 1 to 10 moles of the basic organic nitrogen compound are used per mole of 3-methylpyridine 1-oxide.

6. Process according to Claim 1 to 5, characterized in that the reaction mixture formed in the reaction is subjected to steam distillation.

7. Process according to Claim 1 to 6, characterized in that a further separation by distillation is carried out to obtain the pure compound (I).

## Revendications

1. Procédé pour fabriquer la 2-chloro-5-méthylpyridine de formule (I) 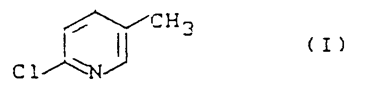 en faisant réagir du 3-méthyl-pyridine-1-oxyde avec du chlorure de phosphoryle,
caractérisé en ce que l'on effectue la réaction en présence d'un composé azoté organique basique de la série comportant les dialkylamines, les trialkylamines, les dialkylcycloalkylamines, les dialkylaralkylamines et les dialkylarylamines et en présence d'un solvant organique inerte à des températures de -50°C à +50°C, et que l'on sépare éventuellement à nouveau le produit réactionnel.

2. Procédé selon la revendication 1, caractérisé en ce que l'on utilise la diisopropylamine comme composé organique azoté.

3. Procédé selon la revendication 1 ou 2, caractérisé en ce que l'on emploie comme solvant organique inerte le chlorure de méthylène.

4. Procédé selon les revendications 1 à 3, caractérisé en ce que la réaction est effectuée à des températures de -20°C à +20°C.

5. Procédé selon les revendications 1 à 4, caractérisé en ce que l'on utilise 1 à 10 moles de composé azoté organique basique par mole de 3-méthylpyridine-1-oxyde.

6. Procédé selon les revendications 1 à 5, caractérisé en ce que l'on soumet le mélange réactionnel produit par la réaction à un entraînement à la vapeur d'eau.

7. Procédé selon les revendications 1 à 6, caractérisé en ce que l'on exécute, pour obtenir le composé (I) pur, une séparation supplémentaire par distillation.
